(19) Europäisches Patentamt
European Patent Office
Office européen des brevets

(11) Publication number: **0 281 204 A2**

(12) **EUROPEAN PATENT APPLICATION**

(21) Application number: **88200386.6**

(51) Int. Cl.⁴: **A61K 9/50**, A61K 9/52

(22) Date of filing: **01.03.88**

(30) Priority: **06.03.87 IT 1961587**

(43) Date of publication of application: **07.09.88 Bulletin 88/36**

(84) Designated Contracting States: **AT BE CH DE ES FR GB GR IT LI LU NL SE**

(71) Applicant: **Dott. Bonapace & C. S.p.A.**
**Via Canova, 12**
**I-20145 Milano(IT)**

(72) Inventor: **Gronchi, Paolo**
**Via Servio Tullio, 4**
**I-20123 Milano(IT)**
Inventor: **Cosentino, Roberto Francesco**
**Via Rucellai, 20/7**
**I-20126 Milano(IT)**

(74) Representative: **Riccardi, Sergio**
**Riccardi & Co. Via Macedonio Melloni, 32**
**I-20129 Milano(IT)**

(54) **Method of protecting scarcely stable substances with polymeric mixture and application thereof.**

(57) The invention relates to methods for the application of a protective film to all types of powders in order to make them more inert to chemical and physical attack. This film is obtained with polymers containing various percentages of Si and with procedures conducted with and without electrical fields in which the operations of granulation and covering the powders and granules are conducted simultaneously. The product is obtained by atomisation of a suspension of the powder in a suitable organic solvent, in which the polymer can be dissolved, obtained with a rotating disk of a suitable material with good electrical conductivity. The resulting product has a different density and greater fluidity and, above all, greater stability than the starting material. Film-forming technologies also permit the design of simpler forms of delayed-dissolution pharmaceuticals and/or masking of the taste. The processes described are suitable for use on production lines of particularly labile products thus reducing to a minimum the difficulties involved in treating them.

EP 0 281 204 A2

# "METHOD OF PROTECTING SCARCELY STABLE SUBSTANCES WITH POLYMERIC MIXTURES AND APPLICATION THEREOF."

The present invention relates to methods for obtaining films on all types of inorganic, organic, metallic powdered mixtures using polymers of the type.

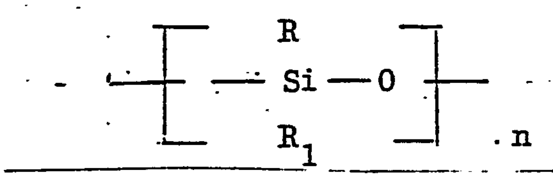

(I)

where $R = R_1$ = alkyl or aryl, or R = alkyl, aryl and $R_1$ = the radical of an organic molecule containing O,N,S or $R = R_1$ = the radical of an organic molecule containing O,N,S, and where n is an integer.

By means of the processes described in the invention it is possible to obtain a complete film of minimal thickness on the surfaces of powders, granulates obtained by the utilisation of the same polymer or others as binders, thus protecting them from attack by atmospheric agents (humidity and/or oxygen), or by other substances with which they come into contact either casually or intentionally, or also to isolate them electrically.

There are in fact many chemical products of synthesis or of biological origin which, because of their nutritional and/or pharmacological characteristics or merely as technological aids, are utilised together with compounds with which they are chemically or physically incompatible, or because these compounds can act as catalysts to accelerate the reactions of the product in question with another component of the mixture or with air. This explains why, then these powders are used in human diets or fed to animals because they have a specific pharmacological action or a specific nutritional value, these powders have often lost most of their active power or their nutritional value at the time they are actually adminstered.

This invention describes film-forming of the active principles with silicon polymers of the (I) type with the aim of extending their stability with time, and also includes the processes of film-forming with and without electrical fields and the single products covered with a film of the aforesaid polymers utilising the procedures here described.

The techniques used today to form films on powdered or liquid substances can be grouped into the following categories: phase separation of an aqueous solution; interface polymerisation, polymerisation in situ, liquid phase solidification by cooling, gelling from a liquid state by chemical reaction, drying from the liquid stage, drying by atomisation, vacuum evaporation. These membrane depositing techniques have found applications for products with a high commercial value but, unlike the present invention, no application has been found in the field of film-forming of products for zootechnical use or pharmaceutical products with a low commercial value because of the high costs of the processes.

The present invention describes methods of covering particles of a size varying from 5 to 1000 m with type (I) polymers having a low surface tension (10-30 dine/cm), soluble in solvents which are highly volatile and have a relatively low boiling point (40-80°C). If the simple operation of adding the reagents is excluded, the film-forming process is a single-stage procedure, at a temperature of less than 50°C with minimum outlay of energy.

The material on which the protective membrane must be constructed is first suspended by means of a simple operation of mixing in a suitable organic solvent, which has good volatility, in which the type (I) polymer or a mixture of type (I) polymers and other functional additives such as molecules with functional groups capable of binding chains of polymers and catalysers for the cross-linking reaction, have previously been mixed in the appropriate quantities to form a film on the surface of the solid material. As a non-limiting example, suitable type (I) polymers are dimethyl polysiloxane and/or diethyl siloxane.

The process is of the physical-chemical type in which the filmforming occurs by the thickening of the filmforming mixture as the solvent evaporates. The whole process takes place on the surface of the rotating disk of an atomiser onto which the suspension is fed. The rotating disk is constructed in such a way as to be as compatible with the phenomena responsible for the process as possible. In fact, in this invention the forming of the film on the particles and the granulation of the powder is an effect correlated to the wettability of the surfaces of the solid particles in suspension, to the wettability of the surfaces of the rotating disk, to the surface tension of the liquid film, to the centrifugal acceleration force and to the electric voltage, when this is applied.

In this case, the application of an electrostatic field at the point of atomisation of the suspension or emulsion solution produces, at certain levels of resistivity, accelerated atomisation by means of the contribution of additional energy to the process of rupturing the liquid threads.

The energy supplied in the form of an electric charge produces an increased state of instability in the liquid phase containing the polymer in the case of solid suspension or in the whole solution or emulsion. Thus the intermolecular phases are decreased and the surface tension is lowered and, as a result, the covering liquid is more disposed to arrange itself in the form of a homogeneous film over the surface of the products to be encapsulated, both when these are represented by solid particles or immiscible liquids in the phase containing the film itself.

Generally, the larger the dimensions of the particles the more they tend to escape from the liquid film which forms on the atomiser, while the finer particles tend to remain trapped in the solvent in which the polymer has been dissolved. All these factors contribute to the correct utilisation of the process for efficient filmforming.

One important element which distinguishes the process described from a normal spray-drying process is that it can be obtained with a choice of appropriate variables such as, if a rotating atomiser is used, the number of revolutions, the diameter of the rotating disk, the feeding speed, the sign and size of the voltage, granulation of the powder, in order to vary its mass density, to economise on the covering polymer and to obtain greater product stability.

The process can be used solely to obtain granulation of the product to obtain physical forms which can be treated, in those cases in which the starting product is pasty, or not flowable or too fine to be used under normal conditions.

The rotating disk is installed at the top of a sealed chamber which is rendered inert by combusted air and/or inert gas which is continuously sucked out, depowdered and partially recycled to the atomiser. As soon as the suspension reaches the rotating plate, the solvent begins to evaporate and a polymer film gradually begins to form around the solid centres. The process generally takes less than one second, depending on the size of the granule, and terminates during the path of the particle through the air. A dry product constituted by product particles of varying dimensions covered by this membrane collects at the bottom of the container. Compared to the particles of the starting product, these particles are glossier, have a lower mass density and greater flowability.

The same results can be obtained with pressure atomisers, with two liquid atomisers and rotating disk atomisers.

The product obtained can subsequently be treated thermally (in some cases the process can be carried out at ambiental temperature) in order to obtain a better cross-linking of the polymer membrane to improve the characteristics of resistance to abrasion and obtain lower porosity. The temperature and duration of the thermal treatment also depend on the characteristics of the encapsulated product.

The filmforming technologies described also permit the production of delayed dissolution pharmaceuticals and masking of the taste.

Some examples of filmforming with silicon-based polymer mixtures on substances with low stability are described below. The examples given are non-limiting and are only given to provide a better illustration of the scope of this invention.

EXAMPLE 1 (Menadione sodium bisulphite)

900g of liquid paraffin hydrocarbon (boiling point 40°C-70°C) and 500 g of menadione sodium bisulphite (MSB) are put into a 2 litre lined glass reactor. The suspension is maintained in agitation by a paddle mixer (500 r.p.m) and then 77 g of silicon resin, constituted by dimethyl siloxane with a high content of methyl groups (for example, the HKA 15 product sold in Italy by WACKER or a similar product) diluted in a mixture of paraffin and alcohol solvents.

Using a peristaltic pump, the suspension is fed to the rotating disk (diameter 100mm) atomiser, installed on the inside face of the upper extremity of a stainless steel cylinder with a diameter of 5000 mm, rotating at a speed of 2000 r.p.m. The feeding speed is such as to stop the deposit of the solid inside the pipes.

Experiments were carried out with and without an electric field; a potential of 50KV is applied to the disk by means of a suitable electric field generator. The completely dry product collects at the base of the spraying cylinder which has a conical shape to facilitate collection.

64.6% of the Menadione sodium bisulphite which is recovered has a granulometry of 250 u m and the remaining 35.4% has a granulometry of 177 to 250 u m (MSB as such: 250 u m 1.3%; 177-250 um 98.7%). The granulometry effect of the process is obvious, while analyses carried out with an electron microscope

show that the granulated particles are uniformly covered with a thin membrane. The product obtained is very fluid, does not pack together under pressure and has an excellent rest angle.

Experiments carried out with electrical fields of different voltages showed that the size of the granulometry decreased as the strength of the electrical field increased and there was a tendency towards monodispersion of the granulometric distribution.

Analysis with an electron microscope showed that there was a greater non-uniform coverage of parts of the same granule with different angles.

EXAMPLE 2 (Ascorbic acid)

1500 ml of petroleum ether and 500 g of ascorbic acid with a granulometry of 50 to 200 u m are placed in a 3 litre glass flask. The suspension is agitated continuously and a solution resin constituted by equal parts of dimethyl polysiloxane and diethyl siloxane in hydrocarbon solvents (contents of dry substances 50%) is added. The suspension is fed into the centre of a rotating plate, installed on the internal face of the upper extremity of a cylindrical container, diameter 4m, height 2.5m, at a speed to 100 ml/min. The rotating disk is driven by a compressed air motor at a speed of 3500 r.p.m.

Experiments were carried out with and without an electrical field; a potential of 40 KV was applied to the disk by means of a suitable electrical field generator.

Fine atomisation of the ascorbic acid was obtained and the dry ascorbic acid collected on the bottom of the container. The product was a white and flowing powder. Examination under an electron microscope showed that the particles were uniformly covered with a thin membrane. Membrane deposit efficiency was 84%

EXAMPLE 3 (Nicotinamide).

500 g of nicotinamide and 1100 ml of acetone were placed in a 2 litre glass flask. The suspension was agitated and 750 g of a 50% solution in acetone of a silicon monomer with methyl groups and olefine groups bound to the Si atom. The solution was fed onto a rotating disk atomiser at a speed of 50 ml/min. The disk had a radius of 100 mm and rotated at a speed of 2000 r.p.m.

Experiments were carried out in the presence of an electrical field (field potential 50 KV) applied to the rotating disk, made from a conductive material, and in the absence of an electrical field. The product collects on the lower base of an inverted conic section.

Analysis under an electron microscope shown greater surface coverage and marked angularity of the crystals.

EXAMPLE 4

500 g of CARBADOX containing approximately 20% humidity was mixed with 230 g of acetone and 20 g of polydimethyl siloxane dissolved in acetone to 50% in weight was added. The starting product is not completely soluble in the organic solvent.

The suspension, continuously agitated by means of a paddle agitator, was fed to rotating disk atomiser by means of a peristaltic pump. A 20 KV electric field was applied to the rotating disk which was kept at a constant rotation speed of 3000 r.p.m.

A product with the following granulometry was obtained: 3.4% with a mesh of less than 60, 25% of a 60 to 80 mesh, 53% between 80 and 100 mesh and 18.6% more than 100 mesh. Therefore, according to USP XXI, the product can be identified as FINE.

The products covered with a film of silicon-based polymer mixtures according to the process described in the invention with and without the application of an electrical field were subjected to the same stability tests as the starting product.

The stability tests were carried out by keeping the samples in a chamber for a certain period of time under conditions of controlled temperature and humidity. Test samples were taken at the start and at various intervals of time and the active principle was analysed using methods described in official pharmacopeia or described in the scientific literature on this subject. As an example, the table below gives the stability of menadione sodium bisulphite in powder and covered with a silicon membrane in accelerated tests.

TABLE - STABILITY IN A CHAMBER AT +40°C AND HUMIDITY.

| | | % Degradation after | |
|---|---|---|---|
| Product | Base | 22 Days | 60 Days |
| Menadione sodium bisulphite | 0 | -11.2 | -39.6 |
| Film-covered Menadione sodium bisulphite | 0 | -6.4 | -7.4 |

Obviously, numerous modifications, variations, additions and/or substitutions can be made to the single operations and components of the described process and methods while still remaining within the spirit and objects of this invention and still remaining within its scope of protection as defined in the appended claims.

**Claims**

1. Method for covering substances with limited stability with a protective film characterised by the fact that the protective film is constituted by a mixture of polymers of the type

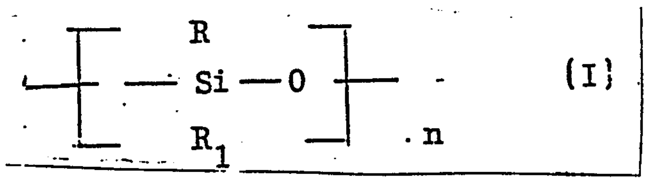

(I)

where R = R, = alkyl or aryl, or R = alkyl or aryl and R, = a radical of an organic molecule containing O,N,S and where n is an integer, and polymers of type (I) can be used either alone or mixed with other polymers, synthesised or natural or with plasticisers and additives.

2. Covering and granulation, micrograngulation and/or microencapsulation method according to claim 1, in which the substance to be covered is kept in suspension in solvents, either pure or in a mixture, in which the film-forming substance has been dissolved and the solution is maintained in agitation by means of a suitable system and continuously fed to a rotating disk atomiser, or a pressure atomiser or a two-fluid atomiser with or without electric field generating equipment.

3. Method according to one or more of the preceding claims characterised by the fact that type (I) polymers are constituted by dimethyl poly siloxane and/or diethyl siloxane.

4. Method according to claims 1 to 3, in which the substances to be covered is menadione sodium bisulphite.

5. Method according to claims 1 to 4, in which the substance to be covered is ascorbic acid.

6. Method according to claims 1 to 4, in which the substance to be covered is nicotinamide.

7. Covering and granulation, microgranulation and/or microencapsulation method according to claims 1 and 2, in which rotating disk atomisation is carried out in order to stabilise the powdered substance.

8. Covering and granulation, microgranulation and/or microencapsulation method according to claims 1 and 2, in which rotating disk atomisation is carried out to obtain pharmaceutical forms and delayed dissolution.

9. Covering and granulation, microgranulation and/or microencapsulation method according to claims 1 and 2, in which rotating disk atomisation is carried out to obtain masking of tastes and smells.

10. Covering and granulation, microgranulation and/or microencapsulation method according to claims 8, 9 or 10 in which an electrical field is applied to the rotating disk to obtain atomisation and the effects indicated.

11. Protective membrane constituted by wax, fats or other products dissolved in organic or aqueous solutions when these are electrostatically sprayed to obtain protective films on fine powders or liquids.

12. Substances covered with protective films by means of atomisation, with or without the application of an electrical field, obtained each time by a method according to one or more of the preceding claims.

13. Process for the protection of substances with limited stability here above and illustrated in the attached examples.